# FASCICULE DE BREVET EUROPEEN

(11) **EP 3 253 731 B1**
(45) Date de publication et mention de la délivrance du brevet: **26.12.2018**
(21) Numéro de dépôt: 16707172.9
(22) Date de dépôt: 28.01.2016
(51) Int. Cl.: C07C 67/03, C07C 67/60, C07C 67/327, C07C 69/54, C07C 213/06, C07C 213/10, C07C 219/08

(54) **VALORISATION DE PRODUITS NOBLES DANS UN PROCEDE DE PRODUCTION D'ESTER (METH)ACRYLIQUE**
RÜCKGEWINNUNG VON EDLEN PRODUKTEN IN EINEM VERFAHREN ZUR HERSTELLUNG VON (METH)ACRYLESTER
RECLAMATION OF NOBLE PRODUCTS IN A METHOD FOR PRODUCING (METH)ACRYLIC ESTER

(30) Priorité: 04.02.2015 FR 1550844
(43) Date de publication de la demande: 13.12.2017
(73) Titulaire: Arkema France, 92700 Colombes (FR)
(72) Inventeur: CABON, Yves, 57000 Metz (FR); DUBUT, Fanny, 57070 Metz (FR); RIFLADE, Benoit, 33430 Bazas (FR)
(74) Mandataire: Bonnel, Claudine
(86) Numéro de dépôt international: PCT/FR2016/050177
(87) Numéro de publication internationale: WO 2016/124837

(56) Documents cités:
- WO-A1-2013/045786
- DATABASE CA [Online] CHEMICAL ABSTRACTS SERVICE, COLUMBUS, OHIO, US; FUKUI, TOMOKI ET AL: "Manufacture of (meth)acrylic acid esters with reduced low-boiling impurities", XP002751652, extrait de STN Database accession no. 2005:51020 -& JP 2005 015398 A (MITSUBISHI RAYON CO) 20 janvier 2005 (2005-01-20)

## Description

### Domaine technique

La présente invention concerne la fabrication d'ester (méth)acrylique selon un procédé en continu par transestérification, et en particulier la fabrication d'acrylate de N,N-diméthylaminoéthyle (désigné ci-après par ADAME).

L'invention fournit un procédé pour produire un ester (méth)acrylique avec une productivité améliorée, par transestérification d'un (méth)acrylate d'alkyle léger, avec un alcool lourd. Le procédé de l'invention inclut le recyclage de produits nobles récupérés après traitement thermique de fractions lourdes générées lors de la synthèse, ledit traitement thermique étant réalisé en présence d'un phtalate de dialkyle dont la chaine alkyle correspond à celle du (méth)acrylate d'alkyle léger.

### Art antérieur et problème technique

Le bilan économique des procédés industriels de production de dérivés (méth)acryliques est fortement lié au recyclage de fractions générées lors du processus de purification des produits bruts, ces fractions étant susceptibles de contenir des réactifs non réagis, des produits secondaires valorisables et/ou le composé recherché, en quantité non négligeable, ainsi que le catalyseur de réaction.

Les procédés par transestérification mettent en jeu un (méth)acrylate d'alkyle à chaine « courte » en C₁-C₄, dit (méth)acrylate d'alkyle léger ou (méth)acrylate léger, qui réagit sur un alcool à chaine carbonée plus « longue », dit alcool lourd, en présence généralement d'un catalyseur et d'inhibiteurs de polymérisation, selon la formule (1) générale suivante :

H₂C=C(R)COOR₁ + R₂OH ⇆ H₂C=C(R)COOR₂ + R₁OH (1)

avec R = H ou CH₃; R₁ chaine alkyle en C₁-C₄ ; R₂OH alcool lourd

Afin de déplacer l'équilibre vers la formation de (méth)acrylate d'alkyle à chaine « longue », l'alcool léger R₁OH libéré au cours de la réaction est éliminé en continu sous forme d'un azéotrope avec le (méth)acrylate léger. Du fait de la présence d'alcool léger, cet azéotrope est avantageusement recyclé sur l'unité de fabrication du (méth)acrylate léger dont la synthèse est basée sur l'estérification directe de l'acide (méth)acrylique avec l'alcool léger.

La réaction de transestérification de dérivés (méth)acryliques entraine la formation d'impuretés, tels que des adduits de Michael résultant de réactions d'addition de Michael d'une molécule d'alcool (contenant un atome d'hydrogène labile) sur la double liaison de l'ester (méth)acrylique.

Par exemple, dans le cas de la fabrication de l'ADAME par transestérification entre un acrylate léger, tel que l'acrylate de méthyle (AM) ou l'acrylate d'éthyle (AE), et le N, N-diméthyl aminoéthanol (DMAE), l'alcool n'ayant pas encore réagi ou les alcools légers générés au cours de la réaction (méthanol ou éthanol) s'ajoutent à la double liaison de l'ADAME déjà formé ou de l'acrylate léger (AM ou AE) n'ayant pas réagi, pour former des sous-produits lourds d'addition de Michael [DMAE+ADAME] de formule : 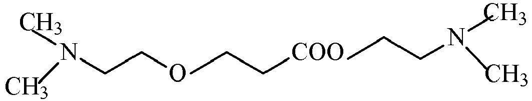 ou [DMAE+AM/AE] de formule :

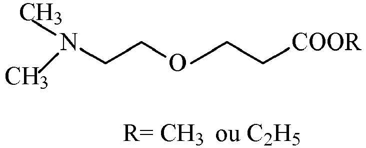

Une caractéristique de ces sous-produits lourds est que leur point d'ébullition se trouve au-dessus des points d'ébullition des produits mis en oeuvre dans la réaction et de l'ADAME recherché.

Ces sous-produits lourds sont généralement concentrés dans une « fraction lourde », séparée au cours du processus de purification de l'ADAME brut, cette fraction lourde pouvant comprendre au sens de la présente invention, non seulement les adduits de Michael, mais aussi généralement le catalyseur de transestérification, les inhibiteurs de polymérisation qui ont été ajoutés à la réaction, ainsi qu'une fraction minoritaire de réactifs résiduels et/ou d'ADAME.

L'élimination de cette fraction lourde pose généralement un problème car elle nécessite d'être incinérée, et entraine des pertes importantes de matières premières (notamment DMAE) et de produit fini (ADAME) qui sont présents dans cette fraction sous forme libre ou sous forme d'adduits de Michael.

Les réactifs résiduels et/ou l'ADAME présents dans la fraction lourde sont des produits nobles puisque leur recyclage permet d'augmenter directement la productivité du procédé. Le recyclage de la totalité de la fraction lourde n'est pas envisageable dans un procédé industriel en continu car il entrainerait une accumulation d'adduits de Michael dans la boucle de purification, à moins d'au préalable craquer thermiquement les adduits de Michael en leurs composants constitutifs.

A cet effet, il a été proposé dans la demande de brevet WO 2013/045786 au nom de la Demanderesse, d'effectuer un craquage thermique des fractions lourdes (méth)acryliques générées lors de la production d'esters (méth)acryliques par transestérification, pour récupérer les produits nobles sous la forme d'un courant de distillat recyclable. Ce procédé se caractérise notamment par l'introduction d'au moins un agent anti-dépôt (ou antifouling) et éventuellement d'un composé abaisseur de viscosité (ou agent fluxant) dans les fractions lourdes avant d'effectuer le craquage thermique de façon à éviter l'encrassement de l'appareillage utilisé et obtenir un résidu ultime suffisamment fluide pour être transporté à l'aide d'une pompe et éliminé par incinération.

Cependant, la Demanderesse a constaté que la présence d'un composé antifouling tel qu'un ester phosphorique, ou d'un composé jouant à la fois le rôle d'agent anti-dépôt et d'agent fluxant, tel que le produit commercialisé par la société Nalco sous la dénomination Nalco® EC3368A, entraine la formation d'impuretés dans le distillat généré lors du craquage thermique.

En particulier, il a été constaté la présence de méthanol en quantité non négligeable dans le procédé de synthèse de l'ADAME à partir d'AE précité utilisant le produit NALCO® EC 3368A comme agent fluxant. La présence de méthanol est particulièrement gênante car le méthanol se retrouve dans les différentes boucles de recyclage, polluant ainsi en tête de réaction l'azéotrope qui est utilisé pour synthétiser au moins en partie l'acrylate d'éthyle, et en conséquence polluant l'acrylate d'éthyle.

De façon surprenante, les inventeurs ont maintenant découvert que l'utilisation d'un phtalate de dialkyle dont la chaine alkyle correspond à celle du (méth)acrylate d'alkyle léger, pour effectuer le craquage thermique de fractions lourdes générées lors de la production d'esters (méth)acryliques par transestérification d'un (méth)acrylate d'alkyle léger avec un alcool lourd, permet non seulement d'éviter l'encrassement de l'appareillage utilisé et d'obtenir un résidu ultime transportable à l'aide d'une pompe pour être incinéré, mais aussi évite les risques de pollution potentielle. Cette utilisation élargit ainsi les possibilités de recyclage des produits nobles valorisables. En outre, de façon inattendue, il a pu être mis en évidence qu'un tel composé a un effet bénéfique sur l'efficacité du craquage thermique.

Un des objectifs de la présente invention est donc de remédier aux inconvénients du procédé précité décrit dans le document WO 2013/045786. La présente invention permet de recycler à différents stades de la section de purification, les produits nobles (composés de départ ou produit fini) potentiellement récupérables dans la fraction lourde générée dans un procédé de synthèse d'esters (méth)acryliques par transestérification d'un (méth)acrylate d'alkyle léger, en particulier dans un procédé de synthèse de (méth)acrylate de dialkylaminoalkyle. Cette valorisation conduit à l'amélioration du bilan matières du procédé et à la réduction des quantités finales de résidu à incinérer, et par conséquent elle représente un avantage économique.

Il est par ailleurs apparu aux inventeurs que la présente invention pouvait également s'appliquer à la production de (méth)acrylate d'alkyle à chaine alkyle linéaire ou ramifiée comportant de 5 à 12 atomes de carbone.

### Résumé de l'invention

La présente invention a donc pour objet un procédé de récupération de produits nobles à partir d'une fraction lourde (méth)acrylique générée lors de la production d'un ester (méth)acrylique par réaction de transestérification d'un (méth)acrylate d'alkyle léger en C₁-C₄, avec un alcool lourd en présence d'un catalyseur, la fraction lourde comprenant au moins des produits nobles et des adduits de Michael résultant de réactions d'addition sur les doubles liaisons (méth)acryliques ainsi que le catalyseur, ledit procédé comprenant le traitement thermique de ladite fraction lourde à une température suffisante pour craquer les adduits de Michael en leurs composants nobles constitutifs, la récupération des produits nobles sous la forme d'un distillat et l'élimination du résidu ultime fluide à l'aide d'une pompe, caractérisé en ce que le traitement thermique est effectué en présence d'au moins un phtalate de dialkyle dont la chaine alkyle correspond à celle du (méth)acrylate d'alkyle léger.

Selon un mode de réalisation de l'invention, au moins une partie de la fraction lourde est recyclée à la réaction de transestérification, l'autre partie étant soumise audit traitement thermique.

Selon un mode de réalisation de l'invention, la fraction lourde est soumise au préalable à une purification par passage sur un évaporateur à film, au moins une partie du flux de pied de l'évaporateur à film étant recyclée à la réaction de transestérification, l'autre partie étant soumise audit traitement thermique. Les composés légers présents dans le flux de tête de l'évaporateur à film peuvent être ainsi avantageusement recyclés.

La fraction lourde contient une quantité importante de catalyseur, pouvant représenter jusqu'à 50% en poids. Le recyclage du catalyseur, encore actif, vers la réaction de transestérification, par l'intermédiaire du recyclage d'au moins une partie de la fraction lourde permet de réduire significativement l'alimentation en catalyseur frais dans le réacteur.

Le terme « (méth)acrylique » signifie acrylique ou méthacrylique ; le terme « (méth)acrylate » signifie acrylate ou méthacrylate.

Par produits nobles, on entend les réactifs non réagis (alcool lourd et (méth)acrylate léger mis en oeuvre dans la réaction de transestérification) et l'ester (méth)acrylique recherché.

Par alcool lourd, on entend un alcool primaire ou secondaire comportant une chaine alkyle linéaire ou ramifiée allant de 4 à 18 atomes de carbone, pouvant être interrompue par un ou plusieurs hétéroatomes tels que N ou O.

La fraction lourde comprend les adduits de Michael et des produits nobles, mais aussi généralement le catalyseur de transestérification et les inhibiteurs de polymérisation qui ont été ajoutés à la réaction.

Selon l'invention, l'addition d'au moins un phtalate de dialkyle dans la fraction lourde permet d'obtenir un taux de craquage thermique amélioré, et minimise la teneur résiduelle en adduit de Michael dans le résidu ultime.

Selon l'invention, associer la nature du phtalate de dialkyle introduit comme agent anti-dépôt/agent fluxant dans l'étape de craquage des adduits de Michael, à la nature du (méth)acrylate d'alkyle léger utilisé comme matière première dans le procédé, évite la génération d'impuretés néfastes pour le recyclage des produits nobles.

Selon un mode de réalisation de l'invention, l'alcool lourd est un aminoalcool de formule (II) :

HO-A - N (R'₂)(R'₃) (II)

dans laquelle
- A est un radical alkylène, linéaire ou ramifié, en C₁-C₅
- R'₂ et R'₃, identiques ou différents l'un de l'autre, représentent chacun un radical alkyle en C₁-C₄.

L'alcool lourd peut être par exemple le N,N-diméthyl aminoéthanol (DMAE), le N,N-diéthyl aminoéthanol, le N,N-diméthyl aminopropanol.

Selon un mode de réalisation préféré de l'invention, l'aminoalcool est le N,N-diméthyl aminoéthanol (DMAE), et l'ester (méth)acrylique est l'acrylate de N,N-diméthyl aminoéthyle (ADAME).

Selon un mode de réalisation de l'invention, l'alcool lourd est un alcool de formule R₂OH dans laquelle R₂ représente une chaine alkyle linéaire ou ramifiée en C₅-C₁₂. L'alcool lourd peut être primaire ou secondaire. L'alcool lourd est par exemple le 2-éthyl hexanol ou le 2-octanol.

Selon un mode de réalisation préféré de l'invention, le (méth)acrylate d'alkyle léger est l'acrylate de méthyle et le phtalate de dialkyle est le phtalate de diméthyle.

Selon un mode de réalisation préféré de l'invention, le (méth)acrylate d'alkyle léger est l'acrylate d'éthyle et le phtalate de dialkyle est le phtalate de diéthyle.

Selon un mode de réalisation préféré de l'invention, le (méth)acrylate d'alkyle léger est l'acrylate de butyle et le phtalate de dialkyle est le phtalate de dibutyle.

Un second objet de l'invention est un procédé de fabrication d'un ester (méth)acrylique par réaction de transestérification entre un (méth)acrylate d'alkyle léger en C₁-C₄, avec un alcool lourd,
ledit procédé comprenant au moins les étapes suivantes :
a) la soumission d'un mélange réactionnel comprenant un (méth)acrylate d'alkyle léger, un alcool lourd, un catalyseur de transestérification, et au moins un inhibiteur de polymérisation, à des conditions de transestérification pour former i) un mélange de produits comprenant l'ester (méth)acrylique, le (méth)acrylate d'alkyle léger et l'alcool lourd non réagis, le catalyseur, les inhibiteurs de polymérisation, des adduits de Michael résultant de réactions d'addition sur les doubles liaisons (méth)acryliques, et autres composés lourds tels que oligomères ou polymères ; et ii) un mélange azéotropique (méth)acrylate d'alkyle léger / alcool léger libéré ;
b) la distillation du mélange i) de produits pour récupérer en tête un flux composé essentiellement de l'ester (méth)acrylique recherché et des produits légers, comportant une fraction minoritaire d'adduits de Michael, de produits lourds, et d'inhibiteurs de polymérisation, mais exempt ou sensiblement exempt de catalyseur, et pour laisser en pied une fraction lourde comprenant le catalyseur, les inhibiteurs de polymérisation, les adduits de Michael et les composés lourds, avec une fraction minoritaire de l'ester (méth)acrylique recherché et d'alcool lourd et des traces de produits légers ;
c) la purification du flux de tête permettant d'obtenir l'ester (méth)acrylique purifié ;
d) la soumission d'au moins une partie de la fraction lourde à un procédé de récupération des produits nobles sous la forme d'un distillat tel que défini précédemment ;
e) le recyclage d'au moins une partie dudit distillat dans au moins une étape choisie parmi l'étape a) de réaction, l'étape b) de distillation et 1 étape c) de purification ;
f) éventuellement le recyclage du mélange azéotropique ii) formé à l'étape a), à l'unité de production du méth)acrylate d'alkyle léger ;
g) éventuellement le recyclage d'au moins une partie du résidu ultime fluide résultant de l'étape d), à l'étape a) de réaction ;
h) incinération du résidu ultime fluide résultant de l'étape d) ;
i) éventuellement le recyclage d'une partie de la fraction lourde à l'étape a) de réaction.

Selon un mode de réalisation de l'invention, l'étape de purification c) est réalisée à l'aide de deux colonnes de distillation en série et au moins une partie du distillat issu de l'étape d) est recyclée en tête de la première colonne de purification.

Selon un mode de réalisation de l'invention, la fraction lourde est soumise au préalable, au moins en partie, à une purification par passage sur un évaporateur à film avant l'étape d).

Selon un mode de réalisation, une partie du flux de pied de l'évaporateur à film est recyclée à l'étape a) de réaction.

L'invention est avantageusement mise en oeuvre pour la production de l'acrylate de N,N-diméthyl aminoéthyle (ADAME) par réaction de transestérification entre l'acrylate d'éthyle (AE) et le N,N-diméthyl aminoéthanol (DMAE), l'étape d) étant effectuée en présence de phtalate de diéthyle.

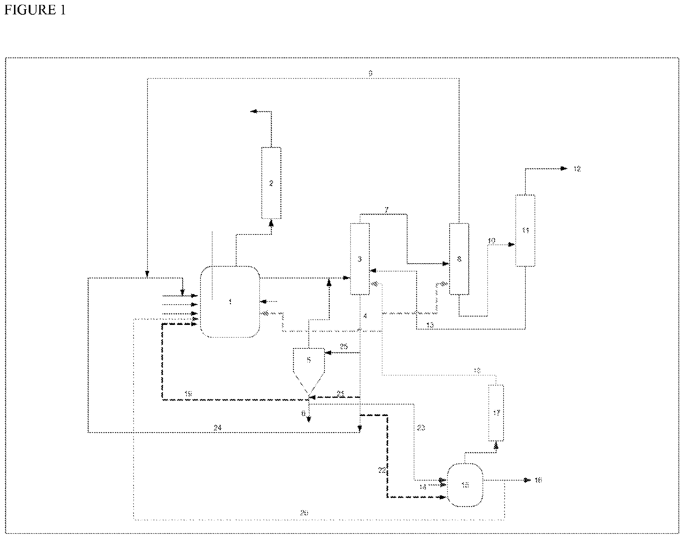

L'invention est maintenant décrite plus en détail et de façon non limitative dans la description qui suit et en référence à la figure 1 annexée qui représente de manière schématique différents modes de réalisation selon l'invention dans une installation pour un procédé continu de production d'ADAME par transestérification à partir d'AE et de DMAE.

### Exposé détaillé de l'invention

Dans le procédé selon l'invention, le traitement thermique de la fraction lourde est effectué en présence d'au moins un phtalate de dialkyle en C₁-C₄, et la chaine alkyle est semblable à la chaine alkyle du (méth)acrylate d'alkyle léger utilisé comme matière première pour produire l'ester (méth)acrylique.

Selon un mode de réalisation de l'invention, le (méth)acrylate d'alkyle léger est l'acrylate d'éthyle (AE), l'alcool lourd est le N,N-diméthyl aminoéthanol (DMAE), l'ester (méth)acrylique est l'acrylate de N,N-diméthyl aminoéthyle (ADAME), et le phtalate de dialkyle est le phtalate de diéthyle.

Selon un mode de réalisation de l'invention, le (méth)acrylate d'alkyle léger est l'acrylate de méthyle (AM), l'alcool lourd est le N,N-diméthyl aminoéthanol (DMAE), l'ester (méth)acrylique est l'acrylate de N,N-diméthyl aminoéthyle (ADAME), et le phtalate de dialkyle est le phtalate de diméthyle.

Selon un mode de réalisation de l'invention, le (méth)acrylate d'alkyle léger est l'acrylate de butyle (ABu), l'alcool lourd est le N,N-diméthyl aminoéthanol (DMAE), l'ester (méth)acrylique est l'acrylate de N,N-diméthyl aminoéthyle (ADAME), et le phtalate de dialkyle est le phtalate de dibutyle.

Selon un mode de réalisation de l'invention, le (méth)acrylate d'alkyle léger est l'acrylate de méthyle (AM), l'alcool lourd est le 2-éthyl hexanol, l'ester (méth)acrylique est l'acrylate de 2-éthyl hexyle (A2EH), et le phtalate de dialkyle est le phtalate de diméthyle.

Selon un mode de réalisation de l'invention, le (méth)acrylate d'alkyle léger est l'acrylate d'éthyle (AE), l'alcool lourd est le 2-éthyl hexanol, l'ester (méth)acrylique est l'acrylate de 2-éthyl hexyle (A2EH), et le phtalate de dialkyle est le phtalate de diéthyle.

Selon un mode de réalisation de l'invention, le (méth)acrylate d'alkyle léger est l'acrylate de butyle (ABu), l'alcool lourd est le 2-éthyl hexanol, l'ester (méth)acrylique est l'acrylate de 2-éthyl hexyle (A2EH), et le phtalate de dialkyle est le phtalate de dibutyle.

Selon un mode de réalisation de l'invention, le (méth)acrylate d'alkyle léger est l'acrylate de méthyle (AM), l'alcool lourd est le 2-octanol, l'ester (méth)acrylique est l'acrylate de 2-octyle (A2OCT), et le phtalate de dialkyle est le phtalate de diméthyle.

Selon un mode de réalisation de l'invention, le (méth)acrylate d'alkyle léger est l'acrylate d'éthyle (AE), l'alcool lourd est le 2-octanol, l'ester (méth)acrylique est l'acrylate de 2-octyle (A2OCT), et le phtalate de dialkyle est le phtalate de diéthyle.

Selon un mode de réalisation de l'invention, le (méth)acrylate d'alkyle léger est l'acrylate de butyle (ABu), l'alcool lourd est le 2-octanol, l'ester (méth)acrylique est l'acrylate de 2-octyle (A2OCT), et le phtalate de dialkyle est le phtalate de dibutyle.

Le phtalate de dialkyle peut être introduit tel quel dans la fraction lourde au niveau du réacteur de craquage, ou introduit en solution dans un solvant, ou encore en solution dans un des réactifs du procédé.

Le phtalate de dialkyle peut être introduit à une concentration allant de 0,001 % à 1 % en poids, notamment de 0,01 à 0,5% en poids, de préférence de 0,1 à 0,5 % en poids dans la fraction lourde à traiter.

Le phtalate de dialkyle présente l'avantage d'agir à la fois en tant qu'agent anti-dépôt (antifouling) et abaisseur de viscosité (agent fluxant) dans l'étape de traitement thermique. Il en résulte un résidu ultime présentant une viscosité adaptée pour être facilement transportable à l'aide d'une pompe, cette viscosité étant généralement inférieure à 200 cP, de préférence inférieure à 50 cP.

La fraction lourde contient l'essentiel du catalyseur employé pour effectuer la réaction de transestérification.

La fraction lourde à traiter peut contenir divers inhibiteurs de polymérisation parmi lesquels on peut citer la phénothiazine (PTZ), l'hydroquinone (HQ) et ses dérivés tels que l'éther méthylique d'hydroquinone, le 2,6 di-terbutyl-4 méthylphénol (BHT), les composés N-oxyls type 4-hydroxy-2,2,6,6 tétraméthyl pipéridinoxyl (4-OH TEMPO) et leurs mélanges en toutes proportions. Il est possible de rajouter une quantité d'inhibiteur de polymérisation allant de 500 à 5000 ppm avant de soumettre la fraction lourde au traitement thermique.

Le traitement thermique est réalisé à une température allant de 100°C à 250°C, de préférence de 150 à 200°C permettant d'éliminer par distillation les produits nobles présents initialement et les produits nobles qui résultent du craquage thermique des adduits de Michael.

Le traitement thermique est effectué sans ajout supplémentaire de catalyseur dans la fraction lourde à traiter.

Le traitement thermique peut être effectué en batch ou en continu dans un réacteur à double enveloppe ou dans un bouilleur surmonté d'une colonne qui joue surtout le rôle de dévésiculeur afin de limiter la montée des inhibiteurs.

Le temps de séjour est généralement compris entre 30 min et deux heures.

Il peut être avantageux de ne soumettre au traitement thermique qu'une partie de la fraction lourde, et de recycler l'autre partie vers la réaction de transestérification.

Selon ce mode de réalisation, la quantité de catalyseur frais à introduire dans le réacteur de transestérification peut être réduite jusqu'à 50% en poids, sans constater d'augmentation de la concentration en composés lourds dans le réacteur.

De préférence, de 5 à 50%, plus particulièrement de 10 à 30% en poids de la fraction lourde sont recyclés vers la réaction, le reste étant soumis au traitement thermique.

Dans une variante préférée de l'invention, préalablement au traitement thermique, la fraction lourde est envoyée sur un évaporateur à film afin de récupérer et recycler les composés légers présents à l'état de traces.

Selon cette variante, il est également avantageux de ne soumettre au traitement thermique qu'une partie du flux de pied de l'évaporateur à film, et de recycler l'autre partie vers la réaction de transestérification.

De préférence, de 5 à 50%, plus particulièrement de 10 à 30% en poids du flux de pied de l'évaporateur à film sont recyclés vers la réaction, le reste étant soumis au traitement thermique.

A l'issue du traitement thermique, les produits nobles, essentiellement l'ester (méth)acrylique recherché et l'alcool non réagi, sont récupérés sous la forme d'un distillat, après distillation sous atmosphère d'azote ou d'air appauvri à 8% volumique d'oxygène et sous pression réduite, par exemple de 10 à 50 mbar. L'utilisation d'air appauvri est préférée.

Les produits nobles ainsi récupérés sont valorisés en les recyclant dans l'installation, à différentes étapes du procédé, soit au niveau de la réaction, soit au niveau des étapes de purification du produit brut réactionnel.

La température du milieu restant supérieure à 60°C, le résidu ultime est suffisamment fluide pour être transportable directement à l'aide d'une pompe.

Le résidu ultime est riche en catalyseur de transestérification et peut être avantageusement recyclé au moins en partie à l'étape de réaction par transestérification.

De préférence, de 5 à 50%, plus particulièrement de 10 à 20% en poids du résidu ultime sont recyclés vers la réaction, le reste étant finalement éliminé par incinération.

La figure 1 annexée illustre un procédé continu de production d'ADAME par transestérification à partir d'AE et de DMAE, dans lequel les étapes (a) à (i) sont applicables de façon plus générale à la production d'esters (méth)acryliques par transestérification à partir de (méth)acrylates d'alkyle légers en C₁-C₄, et d'alcools lourds définis dans le procédé selon l'invention.

Selon une première étape (a), la réaction de transestérification entre l'AE et le DMAE, est effectuée dans le réacteur 1 en présence d'un catalyseur, préférentiellement du titanate de tétraéthyle, et d'inhibiteurs de polymérisation. Le réacteur 1 est surmonté d'une colonne de distillation 2 qui sert à éliminer l'alcool léger formé (éthanol) au fur et à mesure de sa formation et à déplacer ainsi l'équilibre réactionnel dans le sens de la formation de l'ADAME.

La fraction azéotropique générée lors de la réaction de transestérification est avantageusement recyclée à l'unité de production du (méth)acrylate d'alkyle léger, (étape (f)), puisqu'elle est exempte d'impuretés gênantes susceptibles de former des sous-produits (méth)acryliques.

Selon l'étape (b) du procédé, le mélange réactionnel est soumis à une distillation sur une colonne de distillation (colonne d'équeutage 3). On récupère en tête de la colonne 3, un flux 7 débarrassé de l'essentiel du catalyseur et des inhibiteurs de polymérisation et comportant l'ADAME produit et des composés légers avec une fraction minoritaire d'adduits de Michael et de produits lourds.

On récupère en pied de colonne 3 une fraction lourde 4 comprenant le catalyseur, les inhibiteurs de polymérisation, les adduits de Michael et les composés lourds tels que des oligomères et des polymères avec une fraction minoritaire d'ADAME et de DMAE et des traces de composés légers.

Selon l'étape (c) du procédé, le flux 7 est soumis à une purification qui est réalisée à l'aide de la colonne de distillation 8, dont le flux de tête 9 est recyclé à la réaction, le flux de pied 10 étant dirigé vers une colonne de distillation 11 permettant d'obtenir en tête l'ADAME purifié 12, et en pied un flux 13 riche en inhibiteurs qui est recyclé dans le flux de mélange brut réactionnel alimentant la colonne 3.

Selon l'étape (d) du procédé, la fraction lourde 4 issue du pied de la colonne 3 qui contient notamment le catalyseur est pour partie (flux 22) soumise au procédé selon l'invention de récupération des produits nobles (ADAME et DMAE) dans le réacteur 15, l'autre partie (flux 24) pouvant être recyclée dans le réacteur 1 (étape (i) du procédé).

Au moins une partie (flux 25) de la fraction lourde peut être au préalable concentrée sur un évaporateur à film 5 qui permet de séparer les traces de composés légers qui sont alors recyclés à l'alimentation de la colonne 3. La fraction lourde 6 issue de l'évaporateur contient en général en poids environ 1 à 20 % de DMAE, 10 à 30 % d'ADAME, 10 à 40% d'adduits de Michaël [DMAE - ADAME], le reste étant pour l'essentiel constitué de 10 à 50% en poids de catalyseur et d'inhibiteurs de polymérisation et d'autres sous-produits lourds.

Une partie de ce flux 6 peut être recyclée à la réaction (flux 19) afin de réduire l'alimentation en catalyseur frais.

Une partie de la fraction lourde 6, débarrassée des composés légers, est envoyée dans le réacteur 15 (flux 23) après addition d'un phtalate de dialkyle 14 dans les conditions indiquées précédemment.

En variante, une partie de la fraction lourde 4 (flux 21) peut être mélangée avec le flux de pied de l'évaporateur à film, pour être soumise au moins partiellement au traitement thermique. En l'absence ou en présence d'évaporateur à film, une partie de la fraction lourde peut être éliminée par incinération.

Le réacteur 15 peut être du type réacteur à double enveloppe ou bouilleur surmonté d'une colonne à distiller 17 de faible efficacité (1 à 3 plateaux théoriques) qui fait plutôt office de dévésiculeur.

Dans le réacteur 15, la fraction lourde comprenant les adduits de Michael subit un craquage thermique permettant de récupérer un flux 18 riche en DMAE et ADAME en tête de la colonne 17.

Le craquage thermique réalisé dans les conditions selon l'invention permet de récupérer par simple distillation plus de 80% en poids des produits nobles (ADAME et DMAE) contenus dans la fraction 6 issue de l'évaporateur 5, et d'obtenir un taux de craquage des adduits de Michael de plus de 60% massique, voire plus de 70% massique.

Le flux 18 peut être recyclé, selon l'étape (e) du procédé, au niveau du réacteur de synthèse 1, à l'entrée de la colonne d'étêtage 3, ou à l'entrée de la colonne de purification 8 de l'ADAME ; une association de ces différents recyclages est possible.

Les résidus ultimes 16 à la sortie du réacteur 15, riches en catalyseur et inhibiteurs de polymérisation, peuvent être au moins en partie recyclés dans le réacteur de synthèse 1 (flux 20), le reste étant incinéré, en dernière étape (h).

Il est entendu que le procédé de l'invention peut comporter toute combinaison des différentes variantes décrites.

Les exemples ci-après illustrent la présente invention sans toutefois en limiter la portée.

### EXEMPLES

Sauf indication contraire, les pourcentages sont exprimés en pourcentages massiques. Les abréviations suivantes sont utilisées :
- AE : Acrylate d'éthyle
- DMAE : N,N diméthylaminoéthanol
- ADAME : Acrylate de N,N-diméthyl aminoéthyle
- APA : Adduit de Michaël résultant de l'addition de DMAE sur l'ADAME : [DMAE-ADAME]
- APE : Adduit de Michaël résultant de l'addition de DMAE sur l'AE : [DMAE+AE]

### Exemple 1 (comparatif)

Dans un réacteur en verre agité mécaniquement, chauffé à l'aide d'un chauffe-ballon électrique et surmonté d'une colonne Vigreux avec condenseur, séparateur à vide, recette et piège, on a introduit 300 g d'un résidu lourd provenant d'une synthèse d'ADAME à partir d'AE et DMAE.

La composition massique de ce résidu est la suivante :
DMAE : 15,8% - ADAME : 17,5% - APA : 22,3% - APE : 2,5% - qsp 100% : lourds + catalyseur + inhibiteurs. Ce résidu ne contient pas de méthanol.
5000 ppm du composé Nalco® EC3368A ont été ajoutés au mélange, avant de chauffer le résidu sous agitation et bullage d'azote pendant 90 min à 180°C sous une pression de service de 50 mbar. On a récupéré :
Distillât : 150 g
Résidu ultime : 132 g

La composition massique du distillat est :
DMAE : 22,3%
ADAME : 50,5%
APA: 11,66%
AE : 0,44%
Méthanol : 95 ppm
Autres lourds : qsp 100%

L'encrassement du réacteur est très faible, le résidu ultime est visqueux mais ne se solidifie pas à température ambiante.

### Exemple 2 (selon l'invention)

L'exemple 1 a été reproduit en remplaçant le composé Nalco® EC3368A par du phtalate de diéthyle de Sigma Aldrich.

La suite du traitement est similaire à l'exemple 1.

On a récupéré :
Distillât : 155 g
Résidu ultime : 133 g

La composition massique du distillat est :
DMAE : 23,7%
ADAME : 57,2%
APA : 6,52%
AE : 0,84%
Méthanol : 0 ppm
Autres lourds : qsp 100%

L'encrassement du réacteur est très faible, le résidu ultime est visqueux mais ne se solidifie pas à température ambiante. De plus, l'utilisation du phtalate de diéthyle comme fluxant/dispersant a permis d'éviter la formation de méthanol dans le distillat, qui peut être ainsi recyclé sans générer d'impuretés dans le procédé de synthèse de l'ADAME.

### Exemple 3 (en continu, comparatif)

Dans un réacteur en verre, constitué d'un bouilleur thermosiphon, on a introduit à l'aide d'une pompe à membrane une fraction lourde d'une synthèse ADAME. Le débit d'alimentation est régulé par mesure de la masse de résidu dans le bac. Le bouilleur est chauffé à l'aide d'un bain d'huile double enveloppe d'une puissance de 160W afin de minimiser la température de peau. L'ensemble est calorifugé et la température de chauffe est ajustée pour avoir la température requise dans le bouilleur. En tête de bouilleur, on a ajouté un élément de colonne garni par un élément multiknit faisant office de dévésiculeur.

La fraction de pied a été récupérée par débordement dans le bouilleur puis reprise par pompe pour être dirigée vers une recette.

Les opérations ont été effectuées sous pression réduite (50 mbar) et sous bullage d'azote.

La composition massique de la fraction lourde introduite est la suivante :
DMAE : 5%
ADAME : 21,6%
APA : 35,7%
AE : 0,4%
qsp 100% : lourds + catalyseur + inhibiteurs.

La fraction lourde ne contient pas de méthanol.

5000 ppm du composé Nalco® EC3368A ont été ajoutés au mélange.

Les paramètres de fonctionnement du micropilote sont les suivants :
Débit d'alimentation : 110 g/h
Temps de séjour : 90 min
Pression : 50 mbar
Température bouilleur : 180°C

Après une heure de réaction, on a récupéré 51 g de distillat et 59 g de résidu ultime.

La composition massique du distillat est :
DMAE : 23,3%
ADAME : 66,1%
APA : 0%
AE : 2,9%
Méthanol : 104 ppm

La composition massique du résidu est :
DMAE : 13,2%
ADAME : 2,7%
APA : 29,9%
AE : 0,04%
Absence de méthanol
Lourds+catalyseur+inhibiteurs : qsp 100%

Les bilans massiques de cet essai sont les suivants :
- ADAME : sur 23,8 g présent à l'état libre dans la fraction lourde, on récupère 33,7 g dont une partie provient du craquage thermique de l'APA.
- DMAE : sur 5,5 g présent à l'état libre dans la fraction lourde, on récupère 11,8 g dont une partie provient du craquage thermique de l'APA.
- APA : sur 39,3 g présent dans la fraction lourde, il ne reste que 17,6 g après craquage thermique de l'APA.

Le taux de craquage de l'APA, exprimé par la masse d'APA disparue par craquage par rapport à la masse présente est de l'ordre de 55%.

Le réacteur est parfaitement propre (aucune accroche de solide) et le résidu ultime est parfaitement fluide à chaud.

Cependant une quantité non négligeable de méthanol est présente dans le distillat, ce qui entraîne la présence d'une impureté gênante lors du recyclage du distillat dans le procédé de synthèse.

### Exemple 4 (en continu, selon l'invention)

L'exemple 3 a été reproduit avec une fraction lourde de composition suivante :
DMAE : 10,3%
ADAME : 15,3%
APA: 22,1%
AE : 0,08%
Absence de méthanol
qsp 100% : lourds + catalyseur + inhibiteurs.

Le composé Nalco® EC3368A a été remplacé par du phtalate de diéthyle (5000 ppm).

Les paramètres de fonctionnement du micropilote sont :
Débit d'alimentation : 200 g/h
Temps de séjour : 90 min
Température bouilleur : 180°C

Après une heure de réaction, on a récupéré 125 g de distillat et 75 g de résidu ultime. La composition massique du distillat est :
DMAE : 19,9%
ADAME : 68,2%
APA : 0%
AE : 1,46%
Absence de méthanol

La composition massique du résidu est :
DMAE : 8,9%
ADAME : 0,9%
APA : 8,6%
AE : 0,005%
Absence de méthanol
qsp 100% : lourds + catalyseur + inhibiteurs.

Bilan massique :
- ADAME : sur 30,6 g présent à l'état libre dans la fraction lourde, on récupère 85,25 g dont une partie provient du craquage thermique de l'APA.
- DMAE : sur 20,6 g présent à l'état libre dans la fraction lourde, on récupère 24,8 g dont une partie provient du craquage thermique de l'APA.
- APA : sur 44,2 g présent dans la fraction lourde, il ne reste que 8,7 g après craquage thermique de l'APA.

Dans ces conditions, le taux de craquage de l'APA est de l'ordre de 80%, le réacteur est parfaitement propre (aucune accroche de solide) et le résidu ultime est parfaitement fluide à chaud. De plus, le distillat est exempt de méthanol, ce qui permet de renvoyer avantageusement cette fraction au niveau d'une étape du procédé de synthèse et/ou purification.

L'utilisation du phtalate de diéthyle a permis à la fois d'éviter le renvoi de méthanol dans la fraction de distillat, mais également d'améliorer le taux de craquage des sous-produits lourds.

### Exemple 5 (en continu, selon l'invention)

L'exemple 4 a été reproduit avec les conditions suivantes :
La composition massique de la fraction lourde introduite est la suivante :
DMAE : 16% - ADAME : 14% - APA : 24% - qsp 100% : lourds + catalyseur + inhibiteurs.

5000 ppm de phtalate de diéthyle ont été ajoutés.

Les paramètres de fonctionnement du micropilote sont les suivants :
Débit d'alimentation : 200 g/h
Temps de séjour : 90 min
Pression : 50 mbar
Température bouilleur : 180°C
Taux d'épuisement : 50%

Après une heure de réaction, on a récupéré 100 g de distillat et 100 g de résidu ultime. La composition massique du distillat est :
DMAE : 23,1%
ADAME : 60,5%
APA : 0,06%
AE : 2,5%
APE : 0%

La composition massique du résidu est :
DMAE : 14,5%
ADAME : 1,7%
APA : 9,6%
Lourds+catalyseur+inhibiteurs : qsp 100%

Les bilans massiques mettent en évidence la valorisation de l'ADAME et du DMAE récupérés au cours du procédé selon l'invention :
- ADAME : sur 28,9 g présent à l'état libre dans la fraction lourde, on récupère 61,1 g dont une partie provient du craquage thermique de l'APA.
- DMAE : sur 33,3 g présent à l'état libre dans la fraction lourde, on récupère 23,3 g dont une partie provient du craquage thermique de l'APA.
- APA : sur 48,4 g présent dans la fraction lourde, il ne reste que 9,8 g après craquage thermique de l'APA. Le taux de craquage est de 79,8%.

Le réacteur est propre et le résidu ultime est fluide à chaud.

### Exemple 6 : effet du recyclage du catalyseur présent dans le flux de pied de l'évaporateur à film

Dans un réacteur en verre d'1 litre agité mécaniquement chauffé à l'aide d'une double enveloppe, sont introduits le DMAE (2,73 mol), l'AE (4,7 mol, 1,6 éq molaire), ainsi que 21,84 mmol de Ti (OEt)₄ comme catalyseur de transestérification.

Selon les essais, le catalyseur utilisé est une solution 85/15 massique de Ti(OEt)₄ pur dans du DMAE, ou un mélange de cette solution avec du Ti(OEt)₄ issu d'un flux de lourds provenant du pied d'un évaporateur à film d'une unité industrielle d'ADAME. Le milieu réactionnel est alors chauffé à 110°C pendant 3h, la fraction azéotropique AE/EtOH est soutirée pour déplacer l'équilibre.

Le brut réactionnel est ensuite analysé pour calculer le rendement. L'analyse du brut est réalisée par chromatographie en phase gazeuse.

Le rendement en ADAME formé dans ces conditions est déterminé à partir du nombre de moles d'ADAME produit par rapport au nombre de moles de DMAE introduit.

**Tableau 1**

| Essai | Catalyseur Ti(OEt)₄ dans du DMAE, mmoles de Ti(OEt)₄ | Ti(OEt)₄ venant du recyclage, mmoles de Ti(OEt)₄ | Rendement en ADAME, % |
|---|---|---|---|
| 1 | 21,84 | 0 | 77 |
| 2 | 17,47 | 0 | 71 |
| 3 | 10,92 | 0 | 57 |
| 4 | 17,47 | 4,37 | 76 |
| 5 | 10,92 | 10,92 | 76 |

Les résultats des essais réalisés, rassemblés dans le tableau 1 ci-après, montrent que le recyclage de la fraction lourde séparée sur un évaporateur à film permet d'économiser près de 50% en poids de catalyseur d'estérification à production équivalente.

## Revendications

1. Procédé de récupération de produits nobles à partir d'une fraction lourde (méth)acrylique générée lors de la production d'un ester (méth)acrylique par réaction de transestérification d'un (méth)acrylate d'alkyle léger en C₁-C₄, avec un alcool lourd choisi parmi les alcools primaires ou secondaires comportant une chaine alkyle linéaire ou ramifiée allant de 4 à 18 atomes de carbone pouvant être interrompue par un ou plusieurs hétéroatomes tels que N ou O, en présence d'un catalyseur, la fraction lourde comprenant au moins des produits nobles et des adduits de Michael résultant de réactions d'addition sur les doubles liaisons (méth)acryliques ainsi que le catalyseur, ledit procédé comprenant le traitement thermique de ladite fraction lourde à une température suffisante pour craquer les adduits de Michael en leurs composants nobles constitutifs, la récupération des produits nobles sous la forme d'un distillat et l'élimination du résidu ultime fluide à l'aide d'une pompe, **caractérisé en ce que** le traitement thermique est effectué en présence d'au moins un phtalate de dialkyle dont la chaine alkyle correspond à celle du (méth)acrylate d'alkyle léger.

2. Procédé selon la revendication 1 caractérisé en ce le (méth)acrylate d'alkyle léger est l'acrylate de méthyle et le phtalate de dialkyle est le phtalate de diméthyle.

3. Procédé selon la revendication 1 caractérisé en ce le (méth)acrylate d'alkyle léger est l'acrylate d'éthyle et le phtalate de dialkyle est le phtalate de diéthyle.

4. Procédé selon la revendication 1 caractérisé en ce le (méth)acrylate d'alkyle léger est l'acrylate de butyle et le phtalate de dialkyle est le phtalate de dibutyle.

5. Procédé selon l'une quelconque des revendications précédentes caractérisé en ce le phtalate de dialkyle est ajouté à une concentration allant de 0,001 % à 1 % en poids, notamment de 0,01 à 0,5 % en poids, de préférence de 0,1 à 0,5% en poids dans la fraction lourde à traiter.

6. Procédé selon l'une quelconque des revendications précédentes **caractérisé en ce que** l'alcool lourd est un aminoalcool de formule (II) :
HO-A - N (R'₂)(R'₃) (II)
dans laquelle
- A est un radical alkylène, linéaire ou ramifié, en C₁-C₅
- R'₂ et R'₃, identiques ou différents l'un de l'autre, représentent chacun un radical alkyle en C₁-C₄.

7. Procédé selon la revendication 6 **caractérisé en ce que** l'alcool lourd est le N,N-diméthyl aminoéthanol.

8. Procédé selon l'une quelconque des revendications 1 à 5 **caractérisé en ce que** l'alcool lourd est un alcool de formule R₂OH dans laquelle R₂ représente une chaine alkyle linéaire ou ramifiée en C₅-C₁₂, par exemple le 2-éthyl hexanol ou le 2-octanol.

9. Procédé selon l'une quelconque des revendications précédentes, **caractérisé en ce qu'**au moins une partie de la fraction lourde est recyclée à la réaction de transestérification, l'autre partie étant soumise audit traitement thermique.

10. Procédé selon l'une quelconque des revendications précédentes, **caractérisé en ce que** la fraction lourde est soumise au préalable à une purification par passage sur un évaporateur à film, au moins une partie du flux de pied de l'évaporateur à film étant recyclée à la réaction de transestérification, l'autre partie étant soumise audit traitement thermique.

11. Procédé de fabrication d'un ester (méth)acrylique par réaction de transestérification entre un (méth)acrylate d'alkyle léger en C₁-C₄, avec un alcool lourd choisi parmi les alcools primaires ou secondaires comportant une chaine alkyle linéaire ou ramifiée allant de 4 à 18 atomes de carbone pouvant être interrompue par un ou plusieurs hétéroatomes tels que N ou O,
ledit procédé comprenant au moins les étapes suivantes :
a) la soumission d'un mélange réactionnel comprenant un (méth)acrylate d'alkyle léger, un alcool lourd, un catalyseur de transestérification, et au moins un inhibiteur de polymérisation, à des conditions de transestérification pour former i) un mélange de produits comprenant l'ester (méth)acrylique, le (méth)acrylate d'alkyle léger et l'alcool lourd non réagis, le catalyseur, les inhibiteurs de polymérisation, des adduits de Michael résultant de réactions d'addition sur les doubles liaisons (méth)acryliques, et autres composés lourds tels que oligomères ou polymères ; et ii) un mélange azéotropique (méth)acrylate d'alkyle léger / alcool léger libéré ;
b) la distillation du mélange i) de produits pour récupérer en tête un flux composé essentiellement de l'ester (méth)acrylique recherché et des produits légers, comportant une fraction minoritaire d'adduits de Michael, de produits lourds, et d'inhibiteurs de polymérisation, mais exempt ou sensiblement exempt de catalyseur, et pour laisser en pied une fraction lourde comprenant le catalyseur, les inhibiteurs de polymérisation, les adduits de Michael et les composés lourds, avec une fraction minoritaire de l'ester (méth)acrylique recherché et d'alcool lourd et des traces de produits légers ;
c) la purification du flux de tête permettant d'obtenir l'ester (méth)acrylique purifié ;
d) la soumission d'au moins une partie de la fraction lourde à un procédé de récupération des produits nobles sous la forme d'un distillat tel que défini selon l'une quelconque des revendications 1 à 10 ;
e) le recyclage d'au moins une partie dudit distillat dans au moins une étape choisie parmi l'étape a) de réaction, l'étape b) de distillation et 1 étape c) de purification ;
f) éventuellement le recyclage du mélange azéotropique ii) formé à l'étape a), à l'unité de production du méth)acrylate d'alkyle léger ;
g) éventuellement le recyclage d'au moins une partie du résidu ultime fluide résultant de l'étape d), à l'étape a) de réaction ;
h) incinération du résidu ultime fluide résultant de l'étape d) ;
i) éventuellement le recyclage d'une partie de la fraction lourde à l'étape a) de réaction.

12. Procédé selon la revendication 11 **caractérisé en ce que** ladite fraction lourde est soumise au préalable à une purification par passage sur un évaporateur à film avant l'étape d).

13. Procédé selon la revendication 12 **caractérisé en ce qu'**une partie du flux de pied de l'évaporateur à film est recyclée à l'étape a) de réaction.

14. Procédé selon l'une quelconque des revendications 11 à 13 **caractérisé en ce que** l'étape de purification c) est réalisée à l'aide de deux colonnes de distillation en série et au moins une partie du distillat issu de l'étape d) est recyclée en tête de la première colonne de purification.

15. Procédé selon l'une quelconque des revendications 11 à 14 **caractérisé en ce que** l'ester (méth)acrylique est l'acrylate de N,N-diméthyl aminoéthyle résultant de la réaction de transestérification entre le N,N-diméthyl aminoéthanol et l'acrylate d'éthyle et l'étape d) est réalisée en présence de phtalate de diéthyle.

## Patentansprüche

1. Verfahren zur Zurückgewinnung von Wertprodukten aus einer bei der Herstellung eines (Meth)-acrylsäureesters durch Umesterungsreaktion eines leichten C₁-C₄-Alkyl(meth)acrylats mit einem schweren Alkohol, der aus primären oder sekundären Alkoholen mit mindestens einer linearen oder verzweigten Alkylkette im Bereich von 4 bis 18 Kohlenstoffatomen, die gegebenenfalls durch ein oder mehrere Heteroatome wie N oder 0 unterbrochen ist, ausgewählt wird, in Gegenwart eines Katalysators angefallenen schweren (Meth)acryl-fraktion, wobei die schwere Fraktion mindestens Wertprodukte und Michael-Addukte, die aus Additionsreaktionen an den (Meth)acryl-Doppelbindungen stammen, sowie den Katalysator umfasst, wobei das Verfahren die Wärmebehandlung der schweren Fraktion bei einer zum Cracken der Michael-Produkte in ihre wertvollen Aufbaukomponenten, die Gewinnung der Wertprodukte in Form eines Destillats und die Entfernung des flüssigen letztendlichen Rückstands mit Hilfe einer Pumpe umfasst, **dadurch gekennzeichnet, dass** die Wärmebehandlung in Gegenwart von mindestens einem Dialkylphthalat, dessen Alkylkette derjenigen des leichten Alkyl(meth)acrylats entspricht, durchgeführt wird.

2. Verfahren nach Anspruch 1, **dadurch gekennzeichnet, dass** es sich bei dem leichten Alkyl (meth) acrylat um Methylacrylat handelt und es sich bei dem Dialkylphthalat um Dimethylphthalat handelt.

3. Verfahren nach Anspruch 1, **dadurch gekennzeichnet, dass** es sich bei dem leichten Alkyl(meth)acrylat um Ethylacrylat handelt und es sich bei dem Dialkylphthalat um Diethylphthalat handelt.

4. Verfahren nach Anspruch 1, **dadurch gekennzeichnet, dass** es sich bei dem leichten Alkyl(meth)acrylat um Butylacrylat handelt und es sich bei dem Dialkylphthalat um Dibutylphthalat handelt.

5. Verfahren nach einem der vorhergehenden Ansprüche, **dadurch gekennzeichnet, dass** das Dialkylphthalat in einer Konzentration im Bereich von 0,001 bis 1 Gew.-%, insbesondere von 0,01 bis 5 Gew.-%, vorzugsweise von 0,1 bis 0,5 Gew.-%, in die zu behandelnde schwere Fraktion gegeben wird.

6. Verfahren nach einem der vorhergehenden Ansprüche, **dadurch gekennzeichnet, dass** es sich bei dem schweren Alkohol um einen Aminoalkohol der Formel (II) :
HO-A-N(R'₂) (R'₃) (II)
in der
- A für einen linearen oder verzweigten C₁-C₅-Alkylenrest steht,
- R'₂ und R'₃, die gleich oder voneinander verschieden sind, jeweils für einen C₁-C₄-Alkyl-rest stehen,
handelt.

7. Verfahren nach Anspruch 6, **dadurch gekennzeichnet, dass** es sich bei dem schweren Alkohol um N,N-Dimethylaminoethanol handelt.

8. Verfahren nach einem der Ansprüche 1 bis 5, **dadurch gekennzeichnet, dass** es sich bei dem schweren Alkohol um einen Alkohol der Formel R₂OH, in der R₂ für eine lineare oder verzweigte C₅-C₁₂-Alkylkette steht, beispielsweise 2-Ethylhexanol oder 2-Octanol, handelt.

9. Verfahren nach einem der vorhergehenden Ansprüche, **dadurch gekennzeichnet, dass** mindestens ein Teil der schweren Fraktion in die Umesterungsreaktion zurückgeführt wird, wobei der andere Teil der Wärmebehandlung unterworfen wird.

10. Verfahren nach einem der vorhergehenden Ansprüche, **dadurch gekennzeichnet, dass** die schwere Fraktion vorher durch Passage über einen Filmverdampfer gereinigt wird, wobei mindestens ein Teil des Sumpfstroms aus dem Filmverdampfer in die Umesterungsreaktion zurückgeführt wird, wobei der andere Teil der Wärmebehandlung unterworfen wird.

11. Verfahren zur Herstellung eines (Meth)acrylsäureesters durch Umesterungsreaktion zwischen einem leichten C₁-C₄-Alkyl(meth)acrylat und einem schweren Alkohol, der aus primären oder sekundären Alkoholen mit mindestens einer linearen oder verzweigten Alkylkette im Bereich von 4 bis 18 Kohlenstoffatomen, die gegebenenfalls durch ein oder mehrere Heteroatome wie N oder 0 unterbrochen ist, ausgewählt wird, wobei man bei dem Verfahren mindestens:
a) eine Reaktionsmischung, die ein leichtes Alkyl-(meth)acrylat, einen schweren Alkohol, einen Umesterungskatalysator und mindestens einen Polymerisationsinhibitor umfasst, Umesterungsbedingungen unterwirft, was i) ein Gemisch von Produkten, das den (Meth)acrylsäureester und das nicht umgesetzte leichte Alkyl(meth)acrylat und den nicht umgesetzten schweren Alkohol, den Katalysator, die Polymerisationsinhibitoren, Michael-Addukte, die aus Additionsreaktionen an den (Meth)acryl-Doppelbindungen stammen, und andere schwere Produkte wie Oligomere oder Polymere umfasst; und ii) ein azeotropes Gemisch von leichtem Alkyl(meth)acrylat und freiem leichtem Alkohol ergibt;
b) das Gemisch i) von Produkten destilliert, wobei am Kopf ein Strom, der im Wesentlichen aus dem gewünschten (Meth)acrylsäureester und leichten Produkten besteht und eine untergeordnete Fraktion von Michael-Addukten, schweren Produkten und Polymerisationsinhibitoren enthält, aber frei oder weitgehend frei von Katalysator ist, zurückgewonnen wird und am Sumpf eine schwere Fraktion, die Katalysator, Polymerisationsinhibitoren, Michael-Addukte und schwere Verbindungen umfasst und eine untergeordnete Fraktion des gewünschten (Meth)acrylsäureesters und des schweren Alkohols und Spuren von leichten Produkten enthält, zurückbleibt;
c) den Kopfstrom reinigt, wodurch der gereinigte (Meth)acrylsäureester erhalten werden kann;
d) mindestens einen Teil der schweren Fraktion einem Verfahren zur Zurückgewinnung der Wertprodukte in Form eines Destillats gemäß einem der Ansprüche 1 bis 10 unterwirft;
e) mindestens einen Teil des Destillats in mindestens einen Schritt, der aus dem Umsetzungsschritt a), dem Destillationsschritt b) und dem Reinigungsschritt c) ausgewählt wird, zurückführt;
f) gegebenenfalls das in Schritt a) gebildete azeotrope Gemisch ii) in die Einheit zur Herstellung des leichten Alkyl(meth)acrylats zurückführt;
g) gegebenenfalls mindestens einen Teil des flüssigen letztendlichen Rückstands aus Schritt d) in den Umsetzungsschritt a) zurückführt;
h) den flüssigen letztendlichen Rückstand aus Schritt d) verbrennt;
i) gegebenenfalls einen Teil der schweren Fraktion in den Umsetzungsschritt a) zurückführt.

12. Verfahren nach Anspruch 11, **dadurch gekennzeichnet, dass** die schwere Fraktion vorher vor Schritt d) durch Passage über einen Filmverdampfer gereinigt wird.

13. Verfahren nach Anspruch 12, **dadurch gekennzeichnet, dass** ein Teil des Sumpfstroms aus dem Filmverdampfer in den Umsetzungsschritt a) zurückgeführt wird.

14. Verfahren nach einem der Ansprüche 11 bis 13, **dadurch gekennzeichnet, dass** der Reinigungsschritt c) mit Hilfe von zwei Destillationssäulen in Reihe durchgeführt wird und mindestens ein Teil des Destillats aus Schritt d) zum Kopf der ersten Reinigungssäule zurückgeführt wird.

15. Verfahren nach einem der Ansprüche 11 bis 14, **dadurch gekennzeichnet, dass** es sich bei dem (Meth)acrylsäureester um N,N-Dimethylaminoethylacrylat aus der Umesterungsreaktion zwischen N,N-Dimethylaminoethanol und Ethylacrylat handelt und Schritt d) in Gegenwart von Diethylphthalat durchgeführt wird.

## Claims

1. A process for recovering valuable products from a heavy (meth)acrylic fraction generated during production of a (meth)acrylic ester by transesterification reaction of a light C₁-C₄ alkyl (meth)acrylate with a heavy alcohol chosen from primary or secondary alcohols comprising a linear or branched alkyl chain ranging from 4 to 18 carbon atoms, possibly being interrupted by one or more heteroatoms such as N or O, in the presence of a catalyst, the heavy fraction comprising at least valuable products and Michael adducts resulting from addition reactions on the (meth)acrylic double bonds and also the catalyst, said process comprising the heat treatment of said heavy fraction at a temperature sufficient to crack the Michael adducts into their constituent valuable components, the recovery of the valuable products in the form of a distillate and the elimination of the fluid final residue by means of a pump, **characterized in that** the heat treatment is carried out in the presence of at least one dialkyl phthalate, the alkyl chain of which corresponds to that of the light alkyl (meth)acrylate.

2. The process as claimed in claim 1, **characterized in that** the light alkyl (meth)acrylate is methyl acrylate and the dialkyl phthalate is dimethyl phthalate.

3. The process as claimed in claim 1, **characterized in that** the light alkyl (meth)acrylate is ethyl acrylate and the dialkyl phthalate is diethyl phthalate.

4. The process as claimed in claim 1, **characterized in that** the light alkyl (meth)acrylate is butyl acrylate and the dialkyl phthalate is dibutyl phthalate.

5. The process as claimed in any one of the preceding claims, **characterized in that** the dialkyl phthalate is added at a concentration ranging from 0.001% to 1% by weight, especially from 0.01% to 5% by weight, preferably from 0.1% to 0.5% by weight into the heavy fraction to be treated.

6. The process as claimed in any one of the preceding claims, **characterized in that** the heavy alcohol is an aminoalcohol of formula (II):
HO-A-N(R'₂)(R'₃) (II)
in which
- A is a linear or branched C₁-C₅ alkylene radical,
- R'₂ and R'₃, which are identical to or different from one another, each represent a C₁-C₄ alkyl radical.

7. The process as claimed in claim 6, **characterized in that** the heavy alcohol is N,N-dimethylaminoethanol.

8. The process as claimed in any one of claims 1 to 5, **characterized in that** the heavy alcohol is an alcohol of formula R₂OH, in which R₂ represents a linear or branched C₅-C₁₂ alkyl chain, for example 2-ethylhexanol or 2-octanol.

9. The process as claimed in any one of the preceding claims, **characterized in that** at least a portion of the heavy fraction is recycled to the transesterification reaction, the other portion being subjected to said heat treatment.

10. The process as claimed in any one of the preceding claims, **characterized in that** the heavy fraction is subjected beforehand to purification by passage over a film evaporator, at least a portion of the bottom stream from the film evaporator being recycled to the transesterification reaction, the other portion being subjected to said heat treatment.

11. A process for producing a (meth)acrylic ester by transesterification reaction between a light C₁-C₄ alkyl (meth)acrylate and a heavy alcohol chosen from primary or secondary alcohols comprising a linear or branched alkyl chain ranging from 4 to 18 carbon atoms, possibly being interrupted by one or more heteroatoms such as N or O, said process comprising at least the following steps:
a) subjecting a reaction mixture, comprising a light alkyl (meth)acrylate, a heavy alcohol, a transesterification catalyst and at least one polymerization inhibitor, to transesterification conditions in order to form i) a mixture of products comprising the (meth)acrylic ester and the unreacted light alkyl (meth)acrylate and heavy alcohol, the catalyst, the polymerization inhibitors, Michael adducts resulting from addition reactions onto the (meth)acrylic double bonds, and other heavy compounds such as oligomers or polymers; and ii) an azeotropic mixture of light alkyl (meth)acrylate/free light alcohol;
b) distilling the mixture i) of products in order to recover, at the top, a stream composed essentially of the desired (meth)acrylic ester and light products, comprising a minority fraction of Michael adducts, heavy products and polymerization inhibitors, but free or substantially free of catalyst, and in order to leave, at the bottom, a heavy fraction comprising catalyst, polymerization inhibitors, Michael adducts and heavy compounds, with a minority fraction of the desired (meth)acrylic ester and heavy alcohol and traces of light products;
c) purifying the top stream, making it possible to obtain the purified (meth)acrylic ester;
d) subjecting at least a portion of the heavy fraction to a process for recovering the valuable products in the form of a distillate as defined in any one of claims 1 to 10;
e) recycling at least a portion of said distillate into at least one step chosen from step a) of reaction, step b) of distillation and step c) of purification;
f) optionally recycling the azeotropic mixture ii) formed in step a), to the light alkyl (meth)acrylate production unit;
g) optionally recycling at least a portion of the fluid final residue resulting from step d), to step a) of reaction;
h) incinerating the fluid final residue resulting from step d);
i) optionally recycling a portion of the heavy fraction to step a) of reaction.

12. The process as claimed in claim 11, **characterized in that** said heavy fraction is subjected beforehand to purification by passage over a film evaporator before step d).

13. The process as claimed in claim 12, **characterized in that** a portion of the bottom stream from the film evaporator is recycled to step a) of reaction.

14. The process as claimed in any one of claims 11 to 13, **characterized in that** the step of purification c) is carried out by means of two distillation columns in series and at least a portion of the distillate resulting from step d) is recycled to the top of the first purification column.

15. The process as claimed in any one of claims 11 to 14, **characterized in that** the (meth)acrylic ester is N,N-dimethylaminoethyl acrylate resulting from the transesterification reaction between N,N-dimethylaminoethanol and ethyl acrylate, and step d) is carried out in the presence of diethyl phthalate.
